# EUROPEAN PATENT APPLICATION

(11) **EP 1 811 769 A1**
(43) Date of publication of application: **25.07.2007**
(21) Application number: 05806178.9
(22) Date of filing: 09.11.2005
(51) Int. Cl.: H04N 5/232, A61B 1/00, A61B 1/04, A61B 5/07, H04N 5/225

(54) **IMAGING DEVICE**

(30) Priority: 10.11.2004 JP 2004326991
(71) Applicant: Olympus Corporation, Tokyo 151-0072 (JP); SANYO ELECTRIC CO., LTD., Moriguchi-shi, Osaka 570-8677 (JP)
(72) Inventor: MORI, Takeshi, c/o Olympus Corporation, Tokyo 151-0072 (JP); HONDA, Takemitsu, c/o Olympus Medical Systems Corp., Tokyo 151-0072 (JP); SHIGEMORI, Toshiaki, c/o Olympus Medical Systems Corp., Tokyo 151-0072 (JP); TANIMOTO, Takashi, c/o Sanyo El. Co., Ltd., Innov. Group, Gifu 503-0195 (JP)
(74) Representative: von Hellfeld, Axel
(86) International application number: PCT/JP2005/020541
(87) International publication number: WO 2006/051817

(57) **Abstract**

The present invention relates to a compact imaging apparatus. A memory in which one-line data is temporarily stored is provided between an analog processing system and a digital processing system. The analog processing system performs a series of processing from obtaining a pixel signal with an image pickup element to conversion of the pixel signal into digital data, and the digital processing system performs signal processing of the digital data into predetermined imaging data. A controller exclusively controls a one-line analog system operation performed by the analog processing system and a one-line digital system operation performed by the digital processing system. The controller operates the analog processing system with a maximum-speed clock which is possessed by the analog processing system, and the controller operates the digital processing system with a clock corresponding to a frequency determined by a bandwidth of a transmission system which transmits the imaging data. The controller turns on power source supply necessary for the operation of the analog processing system in performing the analog system operation f1 of the analog processing system, and the controller turns off the power source supply necessary for the operation of the analog processing system in performing the digital operation f2 of the digital processing system.

## Description

### TECHNICAL FIELD

The present invention relates to a compact imaging apparatus such as a network camera and a capsule endoscope.

### BACKGROUND ART

Recently, network cameras have been widely used as compact imaging apparatuses which can be connected to a network such as the Internet and LAN. The network cameras allow real-time image monitoring of place away from the network and exhibition of the image while its imaging processing is controlled.

On the other hand, recently, a swallowable capsule endoscope makes an appearance in the field of the endoscope. The swallowable capsule endoscope has an imaging function and a radio communication function. After the capsule endoscope is swallowed from a mouth of a patient for the purpose of observation (examination), the capsule endoscope is moved through the insides of body cavities like organs such as a gaster and a small intestine according to peristaltic movement of the organ, and the capsule endoscope sequentially takes images of the organs until naturally discharged from the human body.

The image data which are taken in the body by the capsule endoscope while the capsule endoscope is moved in the body cavity are sequentially transmitted to the outside of the body through the radio communication and stored in a memory installed in a receiving device outside the body. When the patient takes along the receiving device including the radio communication function and the memory function, the patient can freely act even after swallowing the capsule endoscope until the capsule endoscope is discharged. Then, a doctor or a nurse can make a diagnosis based on the organ image displayed on the screen from the image data stored in the memory.

Patent Document 1: Japanese Patent Application Laid-Open No. 2002-345743
Patent Document 2: Japanese Patent No. 3239087

### DISCLOSURE OF INVENTION

### PROBLEM TO BE SOLVED BY THE INVENTION

Downsizing is demanded in the imaging apparatus used in the network camera and capsule endoscope. In the case where radio transmission is performed by the portable imaging apparatus, however, the radio transmission and analog processing require large power consumption, thereby requiring a power supply having a large capacity to supply such a large power. As a result, downsizing of the imaging apparatus is difficult.

It is an object to provide an imaging apparatus which can be downsized by achieving low power consumption.

### MEANS FOR SOLVING PROBLEM

In order to solve the above problem, an imaging apparatus according to one aspect of the invention includes a memory in which data in predetermined processing unit is temporarily stored, the memory being provided between an analog processing system and a digital processing system, the analog processing system performing a series of processing from obtaining a pixel signal with an image pickup element to conversion of the pixel signal into digital data, the digital processing system performing signal processing of the digital data into predetermined imaging data; and a controller which operates the analog processing system with a maximum-speed clock which is possessed by the analog processing system while operating the digital processing system with a clock corresponding to a frequency determined by a bandwidth of a transmission system which transmits the imaging data, the controller exclusively controlling a processing operation in the predetermined processing unit performed by the analog processing system and a processing operation in the predetermined processing unit performed by the digital processing system.

In the imaging apparatus according to the invention, the controller turns on power source supply necessary for the operation of the analog processing system when the analog processing system is operated, and the controller turns off the power source supply necessary for the operation of the analog processing system when the digital processing system is operated.

In the imaging apparatus according to the invention, the memory is a line memory in which one line is temporarily stored, the one line being of a minimum processing unit of the digital processing system.

In the imaging apparatus according to the invention, the controller turns on the power source supply so that clamp is started after a stabilizing period from the time of power source supply on to the analog processing system until the signal voltage stabilization for the power source supply.

The imaging apparatus according to the invention outputs a series of imaging signals by performing predetermined signal processing to a series of image signals obtained by an image pickup element, and performs control in which power source supply is turned on during a processing period of the series of imaging signals, the power source supply is turned off out of the processing time, and the power source supply is turned on in order to start clamp after stabilizing period from the time of the power source supply on until the signal voltage stabilization for the power source supply.

In the imaging apparatus according to the invention, the imaging apparatus is used as a compact imaging module of a network camera.

In the imaging apparatus according to the invention, the imaging apparatus is used as an intra-subject introduction apparatus including a capsule endoscope.

### EFFECT OF THE INVENTION

According to the present invention, a memory in which data in predetermined processing unit is temporarily stored is provided between an analog processing system and a digital processing system, the analog processing system performs a series of processing from obtaining a pixel signal with an image pickup element to conversion of the pixel signal into digital data, and the digital processing system performs signal processing of the digital data into predetermined imaging data. Furthermore, a controller exclusively controls a processing operation in the predetermined processing unit performed by the analog processing system and a processing operation in the predetermined processing unit performed by the digital processing system. The controller operates the analog processing system with a maximum-speed clock which is possessed by the analog processing system, and the controller operates the digital processing system with a clock corresponding to a frequency determined by a bandwidth of a transmission system which transmits the imaging data. The controller turns on power source supply necessary for the operation of the analog processing system in performing the operation of the analog processing system, and the controller turns off the power source supply necessary for the operation of the analog processing system in performing the operation of the digital processing system. Therefore, the invention has the effect of being able to decrease the noise in the imaging signal and of being able to promote the low-electric power consumption.

### BRIEF DESCRIPTION OF DRAWINGS

FIG. 1 is a view showing schematic configuration of an imaging system including a network camera in which an imaging apparatus according to a first embodiment of the invention is used;
FIG. 2 is a block diagram showing a detailed configuration of the imaging apparatus shown in FIG. 1;
FIG. 3 is a timing chart showing a process operation performed by the imaging apparatus shown in FIG. 1;
FIG. 4 is a timing chart showing a failure state at the time of power source supply on and at the time of clamp start of a timing generation circuit;
FIG. 5 is a timing chart showing a good state at the time of power source supply on and at the time of clamp start of the timing generation circuit;
FIG. 6 is a timing chart showing a failure state at the time of power source supply on and at the time of clamp start of a timing generation circuit when one-frame image signal processing is started;
FIG. 7 is a timing chart showing a failure state at the time of power source supply on and at the time of clamp start of a timing generation circuit when one-frame image signal processing is started; and
FIG. 8 is a view showing a schematic configuration of a radio intra-subject information obtaining system including a capsule endoscope in which the imaging apparatus shown in FIG. 1 is used.

### EXPLANATIONS OF LETTERS OR NUMERALS

- 1, 2: Network camera
- 3: Imaging apparatus
- 4: Station
- 5: Portable terminal
- 10: Wireless LAN
- 11: CCD
- 12: CDS circuit
- 13: A/D conversion circuit
- 14: Digital clamp circuit
- 15: Line memory
- 16: Digital processing circuit
- 17: RF circuit
- 21: Switch circuit
- 22: Power supply circuit
- 23: Timing generation circuit
- 31: Subject
- 32: Receiving device
- 32a: Radio unit
- 32b: Main receiving unit
- 33: Capsule endoscope
- 34: Display device
- 35: Portable recording medium
- PC1, PC2: Terminal device
- N: Network

### BEST MODE(S) FOR CARRYING OUT THE INVENTION

Exemplary embodiments of an imaging apparatus will be described below.

### First Embodiment

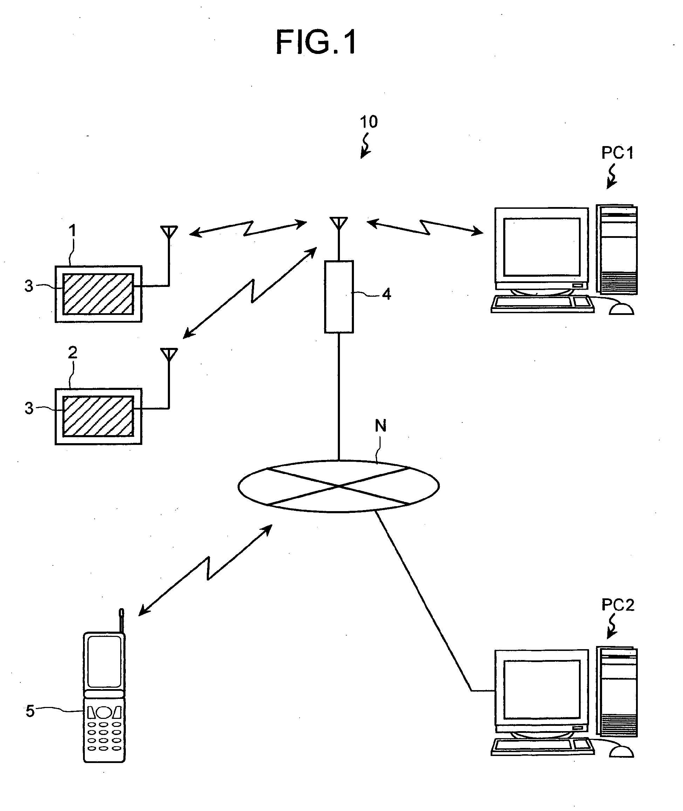

FIG. 1 is a view showing a system configuration in which the network camera as the imaging apparatus is used. As shown in FIG. 1, each of network cameras 1 and 2 includes an imaging apparatus 3 to obtain a video image of the surroundings. The network cameras 1 and 2 are connected to each other and connected to a terminal device PC1 through a wireless LAN station 4 having a router function. The station 4 is connected to a network N such as the Internet, and another terminal device PC2 and a portable terminal 5 can be connected to the network N. For example, the terminal devices PC1 and PC2 can be provided as a personal computer including a display unit, and video information imaged by the network cameras 1 and 2 can be obtained in real time through a wireless LAN 10 or the network N. The portable terminal 5 can also obtain the video information imaged by the network cameras 1 and 2. The terminal devices PC1 and PC2 and the portable terminal 5 can control a change in an imaging visual field of each of the network cameras 1 and 2.

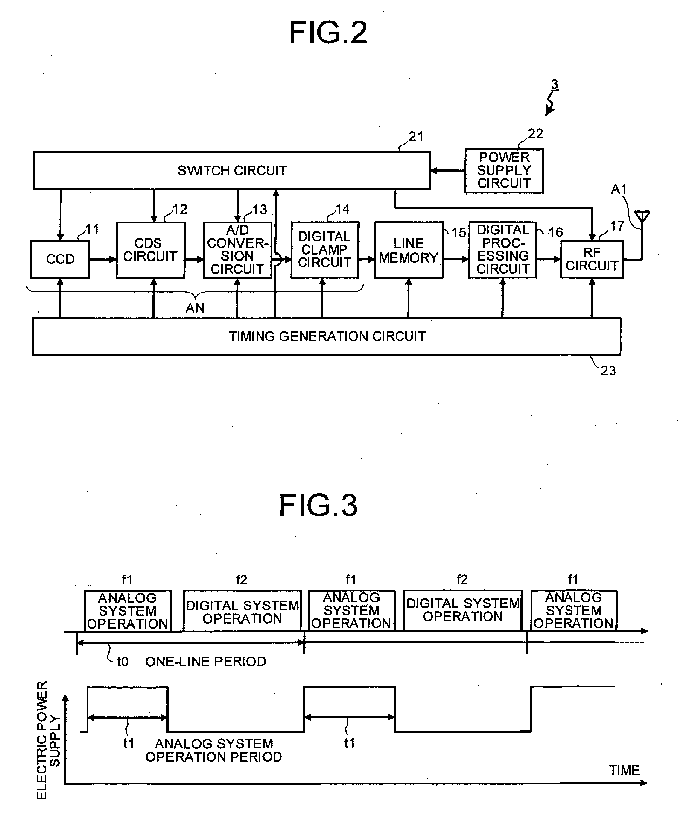

FIG. 2 is a block diagram showing a detailed configuration of the imaging apparatus 3 shown in FIG. 1. Referring to FIG. 2, the imaging apparatus 3 includes an analog processing system AN, a line memory 15, a digital processing circuit 16 as a digital processing system, an RF circuit 17 including an antenna A1, a switch circuit 21, a power supply circuit 22, and a timing generation circuit 23. The analog processing system AN includes a CCD 11, a CDS circuit 12, an A/D conversion circuit 13, and a digital clamp circuit 14. The digital clamp circuit 14 is a circuit which originally performs the processing to the digitized data. However, in the first embodiment, because the signal processing circuit which is of a forestage of the line memory 15 is made the analog processing system AN, the digital clamp circuit 14 should be included in the analog processing system AN. The line memory 15 is provided between the analog processing system AN and the digital processing circuit 16.

The timing generation circuit 23 supplies a clock to the CCD 11, the CDS circuit 12, the A/D conversion circuit 13, the digital clamp circuit 14, the line memory 15, the digital processing circuit 16, the RF circuit 17, and the switch circuit 21, and the timing generation circuit 23 controls the processing of each unit.

A pixel signal imaged by the CCD 11 is output to the CDS circuit 12, and the CDS circuit 12 performs analog processing such as correlated double sampling and gain control to the pixel signal. Then, the pixel signal to which the analog processing is performed is converted into a digital signal by the A/D conversion circuit 13, the digital clamp circuit 14 performs black-level correction processing and the like to the digital signal, and one-line image data is temporarily stored in the line memory 15.

The digital processing circuit 16 takes out the one-line image data stored in the line memory 15, and the digital processing circuit 16 performs the signal processing such as modulation processing. The digital processing circuit 16 performs parallel-serial conversion to the video signal to which the modulation processing has been performed, and the digital processing circuit 16 outputs the converted video signal to the RF circuit 17. The RF circuit 17 performs up-conversion of the input video signal up to a radio frequency, and the RF circuit 17 wirelessly outputs the video signal through the antenna A1.

The switch circuit 21 turns on and off electric power supply to the CCD 11, the CDS circuit 12, and the A/D conversion circuit 13 in the analog processing system AN under the control of the timing generation circuit 23. The switch circuit 21 also turns on and off the electric power supply to the RF circuit 17 under the control of the timing generation circuit 23. In turning on and off the electric power supply to the RF circuit 17, the switch circuit 21 turns on the electric power supply to the RF circuit 17 only when the image information is transmitted.

The control performed by the timing generation circuit 23 will be described with reference to a timing chart shown in FIG. 3. As shown in FIG. 3, in a one-line period t0 during which the imaging apparatus 3 processes the pixel signal imaged by the CCD 11 in each line, the timing generation circuit 23 performs exclusive control in which operation processing of the analog processing system AN and operation processing of the digital processing circuit 16 are temporally separated from each other. In a first half of the one-line period t0, the analog processing system AN is operated to temporarily store the processed one-line image data in the line memory 15 while the line memory 15 becomes a medium. Then, in a last half of the one-line period t0 the digital processing circuit 16 is operated to perform the signal processing to the one-line image data. As shown in FIG. 3, the reason why the electric power supply is increased in the analog system operating period is that bias current flows through the circuit system when the electric power is supplied to the analog processing system AN. An average electric power supply to which time integration is performed can be decreased by shortening the analog system operating period in the analog processing system AN.

In this case, the timing generation circuit 23 sets a clock speed (frequency f1) of the analog processing system AN and a clock speed (frequency f2) of the digital processing circuit 16 under the different conditions, and the timing generation circuit 23 supplies the clock to the analog processing system AN and the digital processing circuit 16. The high-speed clock with which the analog processing system AN can be operated is used for the clock speed of the analog processing system AN, and the high-speed processing is performed. Therefore, an operating time can be shortened in the analog processing system AN. On the other hand, the clock speed of the digital processing circuit 16 is determined by a transmission bandwidth of a radio frequency used in the RF circuit 17. When the transmission bandwidth is narrowed, the clock speed of the digital processing circuit 16 becomes the low speed. When the transmission bandwidth is widened, the high-speed clock can be used. A method of determining the frequency f2 will be described below. Assuming that f0 is a transmission bandwidth of a radio signal, the frequency of the signal input to the RF circuit 17 also depends on a modulation method of the RF circuit 17, and the frequency becomes f0/k, where k is a coefficient determined by the modulation method of the RF circuit 17. Because the input signal of the RF circuit 17 is the output of the digital processing circuit 16, it is necessary that the clock frequency f2 of the digital processing circuit 16 have at least twice as high as the output signal frequency. Therefore, the clock frequency f2 of the digital processing circuit 16 is determined by the relation f2 ≥ 2 × f0/k.

The timing generation circuit 23 also controls the switch circuit 21 to turn on and off the power source supply to the analog processing system AN. As shown in FIG. 3, the timing generation circuit 23 turns on the switch circuit 21 such that the electric power is supplied to the CCD 11, the CDS circuit 12, and the A/D conversion circuit 13 only in the operation period of the analog processing system AN, and the timing generation circuit 23 turns off the switch circuit 21 in other periods. The reason why the on and off control of the electric power supply is performed to the analog processing system AN is that the analog processing system includes many circuits such as a bias power supply in which the large electric power is consumed.

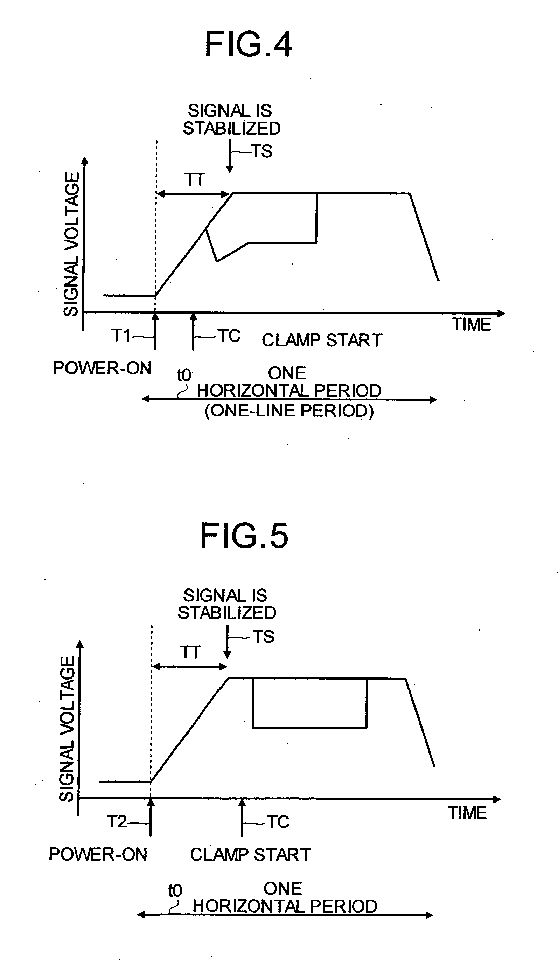

In performing the on and off control of the power source supply, there is a transient period during which the signal voltage rises from the time of power-on to a stable predetermined voltage. Therefore, when clamp processing is performed in the transient period, the signal processing cannot be performed with high accuracy. For example, as shown in FIG. 4, even if the power source supply is turned on at a time point T1, a period TT from the time point T1 to a time point TS is required until the signal voltage is stabilized. On the other hand, the digital clamp circuit 14 starts the clamp at a time point TC in association with the input of the one-line image signal. Accordingly, when the clamp is started at the transient period TT, the image signal becomes deformed.

Therefore, as shown in FIG. 5, the timing generation circuit 23 hastens the time of power source supply on for the analog processing system AN from the time point T1 to the time point T2, and the timing-generation circuit 23 performs the control such that the clamp is started at a time point TC after the period TT elapses from the time point T2. Therefore, the one-line image signal is clamped in the state in which the signal voltage is always stabilized, and the image signal is output with no deformation to the line memory 15.

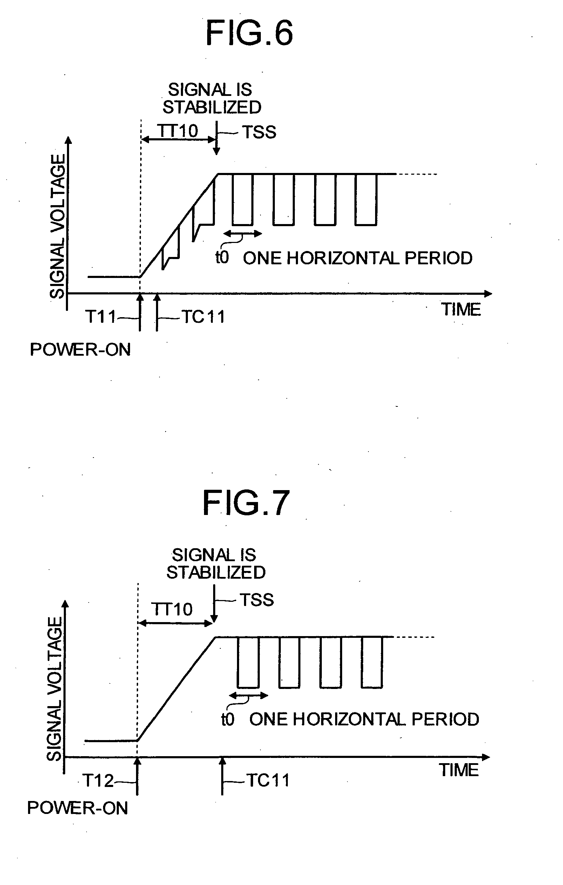

The control at the time of power source supply on shown in FIG. 5 is performed to the one-line image signal. The control can also be applied to the time point control at the time of power source supply on to the conventional analog processing system. In the case where the analog processing system operation processing is performed to the one-frame image signal, as shown in FIG. 6, sometimes several-line image signals are included within a period TT10 from a time point T11 at the time of power source supply on to a time point TSS when the signal voltage is stabilized. In this case, because a time point TC11 when the clamp is started is set immediately before the first line, the first several-line image signals become deformed.

Therefore, as shown in FIG. 7, the time of power source supply on is hastened from the time of time point T11 to the time of time point T12, and the control is performed such that the clamp is started after the period TT10 elapses from the time point T12. Therefore, in the first several-line image signals, the clamp is performed in the state in which the signal voltage is always stabilized, and the image signal is output with no deformation. This state can also be applied to the imaging apparatus 3 shown in FIG. 3. That is, the period TT10 from the time of power source supply on when the one-frame image signal processing is started differs from the period TT from the time of power source supply on in each line. In the case where the power source supply is turned on from the state in which the power source supply is not turned on for a relatively long time, the transient period becomes lengthened. Accordingly, the one-frame image signal processed in the first embodiment can always be output as the image signal having no deformation to the line memory 15 by combining the control during the time of power source supply on shown in FIG. 7 and the control during the time of power source supply on shown in FIG. 5.

In the first embodiment, the line memory 15 is provided between the analog processing system AN and the digital processing circuit 16, the processing operations for the analog processing system AN and the digital processing circuit 16 are temporally separated from each other to perform the exclusive control, and the analog processing system AN and the digital processing circuit 16 differ from each other in the clock speed. Therefore, when the processing operation is performed to the analog processing system AN, the mixture of the noise from the digital processing circuit 16 to the analog processing system AN can securely be prevented to generate the good image information.

In the first embodiment, the clock speed is increased in the analog processing system AN, the operation processing time is shortened in the analog processing system AN, and the electric power is supplied only in the period during which the analog processing system AN is operated. Therefore, the electric power consumption can significantly be decreased in the analog processing system AN, the power supply capacity can be decreased in the power supply circuit, and the downsizing and weight reduction can be promoted in the whole of the imaging apparatus 3.

The timing generation circuit 23 controls the time point during the time of power source supply on such that the clamp processing is performed after the period during which the signal voltage is stabilized from the time of power source supply on. Therefore, the image signal can always be output with no deformation to the line memory 15, which allows the good image information to be generated.

In the first embodiment, it is assumed that the radio transmission is performed. However, the invention can also be applied to the imaging apparatus which transmits the signal by wire. In the first embodiment, it is assumed that the imaging apparatus 3 is battery-driven. However, the invention is not limited to the first embodiment, but the invention can obviously be applied to the imaging apparatus in which commercial power is used. In the first embodiment, the clamp processing is performed with the digital clamp circuit 14. Alternatively, an analog clamp circuit may be provided at the forestage of the A/D conversion circuit 13.

### Second Embodiment

In the first embodiment, the imaging apparatus 3 is applied to the network camera. On the other hand, in the description of a second embodiment, the imaging apparatus 3 is applied to a capsule endoscope.

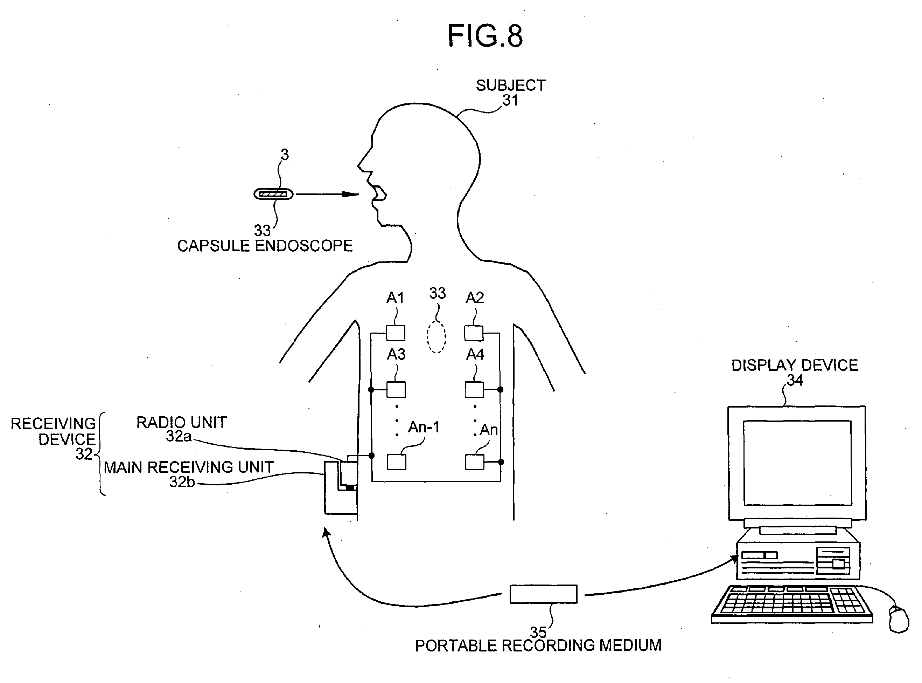

FIG. 8 is a schematic view showing an entire configuration of a radio intra-subject information obtaining system in which a capsule endoscope 33 is used as an example of an intra-subject introduction apparatus. The radio intra-subject information obtaining system includes a capsule endoscope 33, a receiving device 32, a display device 34, and a portable recording medium 35. The capsule endoscope 33 is introduced into a subject 31, and the capsule endoscope 33 takes a body-cavity image to wirelessly perform the transmission of data such as the video signal to the receiving device 32. The receiving device 32 receives the body-cavity image data wirelessly transmitted from the capsule endoscope 33. The display device 34 displays the body-cavity image based on the video image received by the receiving device 32. The portable recording medium 35 is used to perform exchange of the data between the receiving device 32 and display device 34.

The receiving device 32 includes a radio unit 32a and a main receiving unit 32b. The radio unit 32a has plural receiving antennae A1 to An which are adhered to the outer surface of the subject 31. The main receiving unit 32b performs the processing to the radio signal received through the plural receiving antennae A1 to An. These units are detachably connected through a connector or the like. For example, each of the receiving antennae A1 to An is attached to a jacket which the subject 31 can put on, and the subject 31 can attach the receiving antennae A1 to An1 by putting on the jacket. In this case, the receiving antennae A1 to An1 may be detachably attached to the jacket.

The display device 34 displays the body-cavity image taken by the capsule endoscope 33. The display device 34 is realized by a workstation or the like which displays the image based on the data obtained by the portable recording medium 35.

CompactFlash® memory or the like is used as the portable recording medium 35. The portable recording medium 35 can be detachably attached to the main receiving unit 32b and the display device 34, and the portable recording medium 35 has a function of being able to output and record the information in attaching the portable recording medium 35 to the main receiving unit 32b and the display device 34. The portable recording medium 35 is attached to the main receiving unit 32b while the capsule endoscope 33 is moved in the body cavity of the subject 31, and the data transmitted from the capsule endoscope 33 is recorded in the portable recording medium 35. After the capsule endoscope 33 is discharged from the subject 31, namely, after the imaging in the subject 31 is finished, the portable recording medium 35 is taken out from the main receiving unit 32b and attached to the display device 34, and the recorded data is read by the display device 34.

In the second embodiment, the imaging apparatus 3 shown in the first embodiment is incorporated into the capsule endoscope 33. Therefore, the downsizing and weight reduction are further achieved in the capsule endoscope 3, and the good video signal can be transmitted onto the side of the receiving device 32.

### INDUSTRIAL APPLICABILITY

The present invention is useful for the compact imaging apparatus such as the network camera and the capsule endoscope.

## Claims

1. An imaging apparatus comprising:
a memory in which data in predetermined processing unit is temporarily stored, the memory being provided between an analog processing system and a digital processing system, the analog processing system performing a series of processing from obtaining a pixel signal with an image pickup element to conversion of the pixel signal into digital data, the digital processing system performing signal processing of the digital data into predetermined imaging data; and
a controller which operates the analog processing system with a maximum-speed clock which is possessed by the analog processing system while operating the digital processing system with a clock corresponding to a frequency determined by a bandwidth of a transmission system which transmits the imaging data, the controller exclusively controlling a processing operation in the predetermined processing unit performed by the analog processing system and a processing operation in the predetermined processing unit performed by the digital processing system.

2. The imaging apparatus according to claim 1, wherein the controller turns on power source supply necessary for the operation of the analog processing system when the analog processing system is operated, and the controller turns off the power source supply necessary for the operation of the analog processing system when the digital processing system is operated.

3. The imaging apparatus according to claim 1, wherein the memory is a line memory in which one line is temporarily stored, the one line being of a minimum processing unit of the digital processing system.

4. The imaging apparatus according to claim 1, wherein the controller turns on the power source supply so that clamp is started after a stabilizing period from the time of power source supply on to the analog processing system until the signal voltage stabilization for the power source supply.

5. The imaging apparatus according to claim 1, wherein the imaging apparatus is used as a compact imaging module of a network camera.

6. The imaging apparatus according to claim 1, wherein the imaging apparatus is used as an intra-subject introduction apparatus including a capsule endoscope.

7. An imaging apparatus which outputs a series of imaging signals by performing predetermined signal processing to a series of image signals obtained by an image pickup element, wherein the imaging apparatus performs control in which power source supply is turned on during a processing period of the series of imaging signals, the power source supply is turned off out of the processing time, and the power source supply is turned on in order to start clamp after stabilizing period from the time of the power source supply on until the signal voltage stabilization for the power source supply.

8. The imaging apparatus according to claim 7, wherein the imaging apparatus is used as a compact imaging module of a network camera.

9. The imaging apparatus according to claim 7, wherein the imaging apparatus is used as an intra-subject introduction apparatus including a capsule endoscope.
